# EUROPÄISCHE PATENTSCHRIFT

(11) **EP 0 751 753 B1**
(45) Veröffentlichungstag und Bekanntmachung des Hinweises auf die Patenterteilung: **22.10.1997**
(21) Anmeldenummer: 95915117.6
(22) Anmeldetag: 22.03.1995
(51) Int. Cl.: A61F 2/44

(54) **POSITIONIER- UND STÜTZVORRICHTUNG FÜR DIE WIRBELSÄULE**
POSITIONING AND SUPPORT DEVICE FOR THE SPINAL COLUMN
DISPOSITIF DE POSITIONNEMENT ET DE SOUTIEN POUR LA COLONNE VERTEBRALE

(30) Priorität: 23.03.1994 DE 4409939; 16.09.1994 DE 4434384
(43) Veröffentlichungstag der Anmeldung: 08.01.1997
(73) Patentinhaber: Schnorrenberg Chirurgiemechanik GmbH, 16352 Schönwalde (DE)
(72) Erfinder: RUSSEGGER, Lothar, D-15236 Frankurt/Oder Markendorf (DE)
(74) Vertreter: Neumann, Günter
(86) Internationale Anmeldenummer: DE9500443
(87) Internationale Veröffentlichungsnummer: WO9525485

(56) Entgegenhaltungen:
- WO-A-92/01428
- WO-A-92/14423
- DE-A- 3 023 942
- GB-A- 1 243 353
- US-A- 3 750 652

## Beschreibung

Die Erfindung betrifft eine Positionier- und Stützvorrichtung für die Wirbelsäule mit einem im wesentlichen plattenförmigen Trägerelement, welches ventral an aufeinanderfolgenden Wirbeln festlegbar ist.

Die Folge traumatischer Wirbelsäulenerkrankungen sowie degenerativer Prozesse der Wirbelsäule, deren Ursachen Infektionen oder physiologische Degeneration infolge hohen Lebensalters sein können, sind nicht selten pathologische Zustände an Bandscheiben. Derartige Einflüsse können eine irreparable Schädigung einer Bandscheibe oder mehrerer, zwischen benachbarten Wirbelkörpern lokalisierten Bandscheiben, einen sogenannten Bandscheibenprolaps, zur Folge haben. Hierbei ist der Faserring der Bandscheibe eingerissen, wodurch es zum Austritt des weichen Bandscheibenkerns kommt. Die dabei auftretenden Deformationen und Dislokationen des Spinalkanals führen zu einer Kompression des Rückenmarks. Der Patient empfindet dadurch mitunter heftige radikuläre Schmerzen und nicht selten treten neurologische Ausfälle bis hin zur Querschnittslähmung auf.

Bei einer degenerierten Bandscheibe ist als Therapie eine Operation angezeigt. Das operative Vorgehen bei einer cervikalen Bandscheibenoperation ist bei den bisher bekannten Operationsverfahren im wesentlichen durch folgende Schritte gekennzeichnet.
Bei einer Operation im Bereich der Halswirbelsäule wird nach typischem ventralen Zugang und Darstellen der Vorderfläche der Wirbelsäule die vorher unter Röntgenkontrolle identifizierte Bandscheibenhöhe aufgesucht. Die degenerierte bzw. degenerierten benachbarten Bandscheibe(n) wird bzw. werden nach Einschnitt des vorderen Längsbandes entfernt. Der ausgeräumte Zwischenraum wird mittels einer Fräse erweitert. Die nach hinten in Richtung Rückenmark reichenden, als Folge der Instabilität im Segment der geschädigten Bandscheibe meistens vorhandenen Knochenanbauten, auch "spondylotische Randwülste" genannt, werden mit einer Diamantfräse und geeigneten Knochenstanzen abgetragen.

Das Ausräumen der Bandscheibe hat zur Folge, daß die zwischen den benachbarten Wirbeln auftretenden Schub- und Scherkräfte nicht mehr elastisch abgefangen werden. Zur Vermeidung einer Rückenmark- und/oder Wurzelkompression durch voneinander abgleitende oder undefiniert aufeinanderliegende Wirbel werden die benachbarten Wirbelkörper ventral aufgerichtet und in der repositionierten Stellung ventral fusioniert.

Ebenso wie bei Bandscheibenoperationen in nur einem Segment werden auch bei Operationen in zwei oder mehr benachbarten Segmenten die erweiterten Zwischenwirbelräume bei den bekannten Operationsmethoden mit Platzhaltern verschiedenen Materials ausgefüllt. Am weitaus häufigsten gelangt dabei die Methode nach ROBINSON-SMITH zur Anwendung. Bei dieser Methode werden aus dem Knochenkamm des Patienten Knochenchips (kortikospongiöser Span) entnommen, die in die Zwischenwirbelräume eingepfalzt werden. Um eine Lockerung der Knocheninterponate zu verhindern, werden die angrenzenden Wirbelkörper mit einer an der Wirbelsäulen-Vorderfläche angebrachten Metallplatte oder Stützvorrichtung fixiert. Im Falle einer Schädigung von zwei unmittelbar benachbarten Bandscheiben werden demnach 3 Wirbelkörper fusioniert.

Für derartige Operationen sind mehrere Stützvorrichtungen bekannt. Diese Implantate werden in der Regel von dorsal oder von ventral mit den benachbarten Wirbelkörpern verschraubt und sind an Halteschienen oder dergleichen individuell arretierbar. Solche Stützvorrichtungen werden beispielsweise in der EP 0 536 066 A1, der EP 0 348 272 B1 und der US 44 01 112 beschrieben.

Sowohl bei Bandscheibenoperationen in nur einem Segment als auch bei Verwendung der genannten Vorrichtungen zur Durchführung von Plattenosteosynthesen an zwei oder mehr Segmenten der Wirbelsäule besteht ein wesentlicher Nachteil dieser technischen Lösungen darin, daß das Einsetzen von Platzhaltern in die Zwischenwirbelbereiche für den therapeutischen Erfolg unabdingbare Voraussetzung ist. Im Falle des Einbringens von Knochenchips aus dem Beckenkamm des Patienten ist damit ein zusätzlicher operativer Eingriff mit allen damit verbundenen Risiken für den Patienten notwendig. Vor allem bei unfallbedingten traumatischen Schädigungen von Bandscheiben aber auch bei degenerativen Veränderungen der Wirbelsäule infolge höheren Lebensalters stellt eine solche operative Vorgehensweise eine schwere Belastung des Patienten dar.

Bei Einbringen von Palacos-Plomben denaturierter und entsprechend präparierter Fremdknochen, sogenannter "künstlicher Bandscheiben" oder anderer bekannter Stützvorrichtungen in die Zwischenwirbelräume entfällt zwar die Entnahme eines kortikospongiösen Spans aus dem Beckenkamm des Patienten, jedoch ist dies mit der großen Unsicherheit verbunden, daß das eingebrachte Material nicht ausreichend stabil ist, die Palacos-Plombe mit den angrenzenden Wirbelkörpern keine "knöcherne" Verbindung eingeht oder sich die Stützvorrichtung nicht, wie an sich erforderlich, positionieren läßt.
Bei Verwendung eines kortikospongiösen Spans wird außerdem angenommen, daß bei ca. 20 % der Patienten der Span denaturiert, also ebenfalls nicht "knöchert", sondern - soweit er sich nicht auflöst - bestenfalls nur bindegewebig fest wird. Ein biomechanisch instabiles System mit allen damit für den Patienten verbundenen Risiken ist in solchen Fällen die Folge.

Als ein weiteres Problem hat sich erwiesen, daß die kortikospongiösen Späne bzw. die Palacos-Plomben in ihren Abmessungen möglichst genau den Abständen zwischen den benachbarten Wirbelkörpern angepaßt werden müssen. Abgesehen davon, daß das einen besonderen Aufwand erfordert, lassen fehlerhafte Bearbeitungen sich häufig nicht oder nur schwierig korrigieren und beeinträchtigen den therapeutischen Erfolg.

Daraus ergibt sich die Aufgabe der Erfindung, eine gattungsmäßige Positionier- und Stützvorrichtung für Bandscheibenoperationen an zwei und mehr unmittelbar benachbarten Wirbelsäulensegmenten derart weiterzubilden, daß der Umfang der operativen Maßnahmen am Patienten reduziert wird und gleichzeitig eine optimale Anpassung an die individuellen physiologischen Verhältnisse des Patienten erfolgen kann.

Erfindungsgemäß wird diese Aufgabe durch die in dem Anspruch 1 angeführten Merkmale gelöst. Weitere vorteilhafte Ausbildungen des Erfindungsgegenstandes ergeben sich aus den Unteransprüchen 2 bis 12.

Der besondere Vorteil der Erfindung ergibt sich daraus, daß die an einem Trägerelement angebrachten, mit Abstand zueinander angeordneten Stützplatten zum Beispiel bei einer Operation im Bereich der Halswirbelsäule in einem Operationsgang von ventral zwischen die zu fusionierenden, benachbarten Wirbel eingesetzt, positioniert und fixiert werden können. Die Stützplatten befinden sich im implantierten Zustand im Zwischenraum zwischen den fusionierten Wirbelkörpern, der bei Plattenosteosynthesen gemäß dem Stand der Technik in der Regel durch den kortikospongiösen Span des Patienten oder Palacos-Plomben ausgefüllt wird. Dieser Umstand ist besonders vorteilhaft, da zur Repositionierung derartige zusätzliche Mittel nun nicht mehr erforderlich sind.
Das bei Plattenosteosynthesen nach dem Stand der Technik vorhandene Risiko und die damit verbundenen Belastungen des Patienten fallen weg. Außerdem reduzieren sich im Vergleich zu Plattenosteosynthesen unter Verwendung kortikospongiöser Späne sowohl der gesamte operative Aufwand, da die Entnahme von Spongiosa beim Patienten vollständig entfällt, als auch der postoperative Aufenthalt im Krankenhaus.

Weiterhin ist es sehr vorteilhaft, daß der Abstand der Stützplatten voneinander feinfühlig und genau einstellbar ist. Hierdurch wird dem behandelnden Chirurgen die Möglichkeit gegeben, die Stützwirkung der gespreizten Stützplatten auf die zu fusionierenden Wirbelkörper optimal individuell anzupassen. Die Operationstechnik kann im wesentlichen beibehalten werden; es entfallen jedoch die nachteiligen Wirkungen, die aus einer Fehlbearbeitung von bisher verwendeten Spongiosa-Implantaten oder unzureichend positionierten Stützvorrichtungen herrühren. Weil auf Grund der einstellbaren Spreizung keine Vorbearbeitung des Implantats notwendig ist, wird der Arbeitsaufwand beträchtlich reduziert.

Für die geforderte Langzeitstabilität der Positionier- und Stützvorrichtung ist es weiterhin von Vorteil, daß die Stützplatten in gespreizter Stellung fixierbar sind. Auf die Stützplatten der Stützvorrichtung wirken nämlich auf Grund des von der Wirbelsäule abgestützten Körpergewichtes relativ große Kräfte, die insbesondere bei Bewegungen, wie Laufen oder Springen, kurzzeitig hohe Spitzenwerte erreichen. Durch die zusätzliche Fixierung der Stützplatten im gespreizten Zustand wird zuverlässig vermieden, daß diese durch die auftretenden Kräfte zusammengedrückt werden. Eine in diesem Fall sonst notwendige, mit einem operativen Eingriff verbundene Nachjustierung entfällt. Technisch läßt sich eine derartige Fixierung beispielsweise durch eine Blockierung der Spreizvorrichtung oder durch das Einfügen von zusätzlichen Stützkeilen oder unlösbar blockierbaren Schraubstützen realisieren. Ein Nachgeben der gespreizten Stützplatten durch "Losrütteln" ist damit weitgehend ausgeschlossen.

Zur Fusionierung von nur zwei benachbarten Wirbelkörpern kann es für ein sicheres und spannungsfreies Festlegen des Trägerelementes an den Wirbelkörpern vorteilhaft sein, daß das Trägerelement aus gelenkig miteinander verbundenen Teilstücken gebildet wird. In der Regel weisen die miteinander zu fusionierenden Wirbelkörper keine durchgehende, regelmäßige Auflagenfläche auf, an der ein ebenes, gewölbtes oder abgewinkeltes Trägerelement flächig anliegend verschraubt werden könnte. Dadurch, daß die Auflagefläche des Trägerelementes geteilt ist und die Teilstücke durch Scharniere oder dergleichen gelenkig miteinander verbunden sind, sind die einzelnen Teilstücke spannungsfrei auch an unregelmäßig geformten und/oder geneigt zueinander stehenden Wirbelkörper-Außenflächen festlegbar. Dadurch werden die Normal- und Scherbelastungen der Befestigungsschrauben so weit herabgesetzt, daß ein Ausreißen oder eine Beschädigung der Wirbel vermieden wird.

Eine weitere vorteilhafte Ausführungsform der Erfindung sieht vor, daß die Stützplatten gegeneinander kippbar sind. Dadurch wird erreicht, daß der Bearbeitungsaufwand der gegeneinander abzustützenden Wirbelkörper reduziert wird weil eine planparallele Ausrichtung der einander zugewandten Stützflächen der Wirbelkörper nicht notwendig ist. Die gegeneinander kippbaren Stützplatten passen sich in ihrer Lage bei der Verspannung zwischen den Wirbelkörpern von selbst etwaigen Unebenheiten an, so daß die Anpreßkräfte schon bei der Operation gleichmäßig in die benachbarten Wirbelkörper eingeleitet werden. Eine Überbeanspruchung der Wirbelkörper wird somit weitgehend vermieden.

Die erfindungsgemäße Positionier- und Stützvorrichtung ist besonders sicher handhabbar, wenn die Stützplatten einseitig entlang ihrer Bewegungsbahn an dem Trägerelement geführt sind. Je nach den Erfordernissen sind die Stützplatten gegenüber dem Trägerelement linear verschiebbar und/oder kippbar. Durch die Führung befinden sich die Stützplatten entlang ihrer Bewegungsbahn jederzeit in definierten Positionen zueinander und zum Trägerelement. Somit kann die Stützvorrichtung kontrolliert bis in die Endposition zwischen den Wirbelkörpern ausgefahren werden.
Beweglich an dem Trägerelement geführte Stützplatten verhindern das Auftreten mechanischer Spannungen bei der Implantation. Daraus resultiert eine hohe biomechanische Verträglichkeit.

Nach einer vorteilhaften Ausführungsform der Erfindung zur Fusionierung von mehr als zwei unmittelbar benachbarten Wirbelkörpern ist vorgesehen, daß nicht nur der Abstand der Stützplatten voneinander einstellbar ist, sondern unabhängig davon auch der Abstand der Stützplattenpaare eingestellt werden kann. Dem Chirurgen wird dadurch bei Operationen an zwei und mehr Bandscheiben das Einsetzen der Stützplattenpaare in die Zwischenwirbelbereiche erleichtert und zugleich die Möglichkeit gegeben, die Stützwirkung der gespreizten Stützplatten den jeweiligen physiologischen Verhältnissen der mehr als zwei zu fusionierenden Wirbelkörper direkt anzupassen. Bei maximaler Stabilität und ohne die Gefahr der Lockerung des Implantats wird schließlich eine optimale Entlastung der Nervenwurzeln erreicht.

Eine weitere vorteilhafte Ausführungsform der Erfindung sieht vor, daß das Trägerelement aus gelenkig, vorzugsweise mittels Scharnier, miteinander verbundenen Trägerelementen gebildet wird. Die gelenkige Verbindung ist dabei jeweils im Bereich zwischen zwei Stützplattenpaaren an den Trägerelementen angeordnet. Durch diese spezifische gelenkige Ausbildung des Trägerelementes der Positionier- und Stützvorrichtung sind die einzelnen Teilelemente auch an unregelmäßig geformten Wirbelkörpern mit hoher Präzision flächig festlegbar und können ebenso wie die Stützplattenpaare spannungsfrei fixiert und ohne die Gefahr des Ausreißens oder Ausbrechens mit den jeweiligen Wirbelkörpern verschraubt werden.

Gemäß einer weiteren vorteilhaften Ausführungsform der Erfindung ist vorgesehen, daß die Trägerelemente jeweils im Bereich zwischen zwei Stützplattenpaaren, vorzugsweise mittels einer zentralen Befestigungsschraube, fixierbar sind. Damit ist insbesondere die Möglichkeit einer feinfühligen Positionierung der erfindungsgemäßen Vorrichtung in der Vertikalen gegeben.

Als zweckmäßig erweist es sich, daß die Trägerelemente an ihren oberen und unteren Enden jeweils langlochartig ausgebildete Durchbrüche für deren Fixierung mittels Knochenschrauben aufweisen. Damit kann den individuellen physiologischen Verhältnissen des Patienten auch im Hinblick auf unterschiedliche Abstände der Zwischenwirbelkörper zueinander optimal entsprochen werden.

In einer bevorzugten Ausführungsform weist das Trägerelement Führungsschlitze auf, in denen an den Stützplatten anbringbare Zapfen geführt sind. Durch diese Anordnung wird in vorteihafter Weise eine besonders zuverlässige Linearführung der Stützplatten bei der Spreizbewegung erzielt. Weiterhin ergibt sich die Möglichkeit, beispielsweise Schrauben an den Führungszapfen anzubringen, wodurch diese in den Führungsschlitzen festlegbar sind. Damit lassen sich die Stützplatten sicher und mit geringem Arbeits- und Materialaufwand und in gespreizter Stellung absichern, d.h. fixieren.

In einer weiteren bevorzugten Ausführungsform sind die Stützplatten mit Durchbrüchen versehen und/oder weisen eine aufgerauhte Oberfläche auf. Dadurch kann das spongiöse Knochengewebe der durch die Stützplatten gespreizten Wirbelkörper besonders gut mit den Stützplatten verwachsen. Dabei sorgt die aufgerauhte Oberfläche für eine gute Haftwirkung; das durch die Durchbrüche wachsende spongiöse Gewebe ergibt im Laufe der Zeit eine feste mechanische Verbindung zwischen den Stützplatten und den Wirbelkörpern.

Zweckmäßigerweise ist die gesamte erfindungsgemäße Positionier- und Stützvorrichtung aus biologisch verträglichem Material gefertigt, beispielsweise Titan oder Edelstahl. Insbesondere wird sich dann schon nach relativ kurzer Zeit eine feste Verbindung zwischen den Stützplatten und den Wirbelkörpern bilden. Außerdem ist sichergestellt, daß die Stützplatten allen auftretenden Kräften standhalten.

Im folgenden soll die Erfindung anhand von Zeichnungen näher erläutert werden. Es zeigen
- **Fig. 1**: einen vertikalen Schnitt durch eine erfindungsgemäße Positionier- und Stützvorrichtung zur Fusionierung von zwei Wirbelkörpern senkrecht zum Trägerelement mit gespreizten Stützplatten;
- **Fig. 2**: eine erfindungsgemäße Positionier- und Stützvorrichtung in derselben Ansicht wie in **Fig. 1**, mit zusammengezogenen Stützplatten;
- **Fig. 3**: eine Draufsicht auf das Trägerelement gemäß **Fig. 1** bzw. **Fig. 2**;
- **Fig. 4**: eine Ansicht wie **Fig.3** auf ein Trägerelement bestehend aus gelenkig miteinander verbundenen Teilstücken;
- **Fig. 5**: den vertikalen Schnitt durch eine Positionier- und Stützvorrichtung mit zwei gespreizten Stützplattenpaaren entsprechend dem Schnitt A-A in **Fig. 6**
- **Fig. 6**: die Vorderansicht der Positionier- und Stützvorrichtung nach **Fig. 5**
- **Fig. 7**: die Vorderansicht einer Positionier- und Stützvorrichtung mit zwei gelenkig ausgebildeten Trägerelementen
- **Fig. 8**: die Vorderansicht von drei gelenkig miteinander verbundenen Trägerelementen.
- **Fig. 9**: eine mögliche weitere Ausführung der Positionier- und Stützvorrichtung mit mehreren, voneinander beabstandeten Stützplattenpaaren
- **Fig. 10**: den Schnitt B-B aus **Fig. 9**.

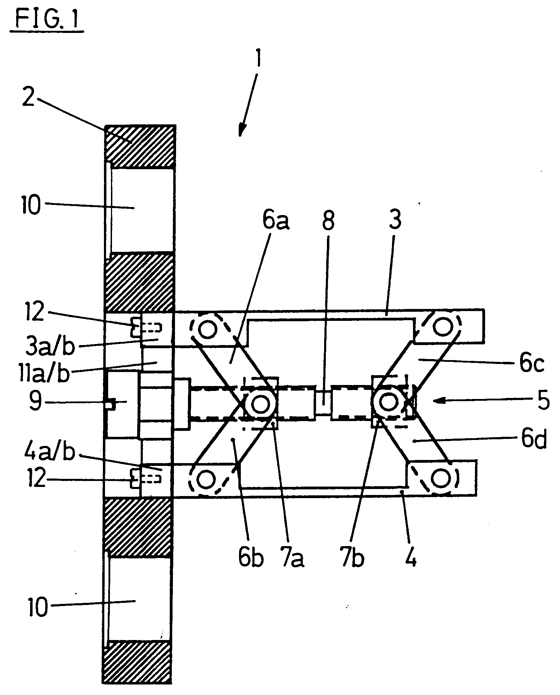

In **Fig. 1** ist die Positionier- und Stützvorrichtung als ganzes mit dem Bezugszeichen 1 versehen. Diese weist eine Trägerelement 2 und Stützplatten 3 und 4 auf, die mit Abstand in etwa parallel zueinander angeordnet sind und von der Trägerelement 2 in der dargestellten Ausführungsform etwa rechtwinklig abstehen.

Die Stützplatten 3 und 4 sind über ein Scherenhubgestänge 5 miteinander verbunden. Das Scherenhubgestänge 5 enthält Kniehebel 6a, b, c und d, Muttern 7a und b sowie eine Betätigungsschraube 8. Die Kniehebel 6a und b sind jeweils an der Stützplatte 3 bzw. 4 angelenkt und in einem an der Mutter 7a angebrachten Kniegelenk miteinander verbunden.

Die Betätigungsschraube 8 ist durch die Muttern 7a und 7b geschraubt. Im Bereich der Mutter 7a ist die Betätigungsschraube 8 mit Rechtsgewinde, im Bereich der Mutter 7b mit Linksgewinde versehen. Dementsprechend weist die Mutter 7a rechts-, die Mutter 7b links-Innengewinde auf.

Die Betätigungsschraube 8 ist in der Trägerelement 2 drehbar gelagert. Der Werkzeugansatz 9 der Betätigungsschraube 8 befindet sich auf der den Stützplatten 3 und 4 abgewandten Betätigungsseite der Trägerelement 2.

Die Trägerelement 2 ist mit mehreren durchgehenden Befestigungsbohrungen 10 versehen.

An der der Trägerelement 2 zugewandten Kante sind die Stützplatten 3 bzw. 4 jeweils mit zwei nebeneinander liegenden Führungszapfen 3a und b bzw. 4a und b versehen. Diese sind längsverschieblich in Führungsschlitzen 11a und b geführt, welche senkrecht verlaufend in die Trägerelement 2 eingebracht sind. In dem dargestellten Schnitt sind nur die Führungszapfen 3a und 4a sowie der als Langloch ausgebildete Führungsschlitz 11a erkennbar.

In die Führungszapfen 3a und b sowie 4a und b sind Fixierungsschrauben 12 eingebaut. Durch Festschrauben dieser Fixierungsschrauben 12 sind die Führungszapfen 3a und b sowie 4a und b und damit die Stützplatten 3 und 4 in den Führungsschlitzen 11a und b, d.h. an der Trägerelement 2 fest verspannbar.

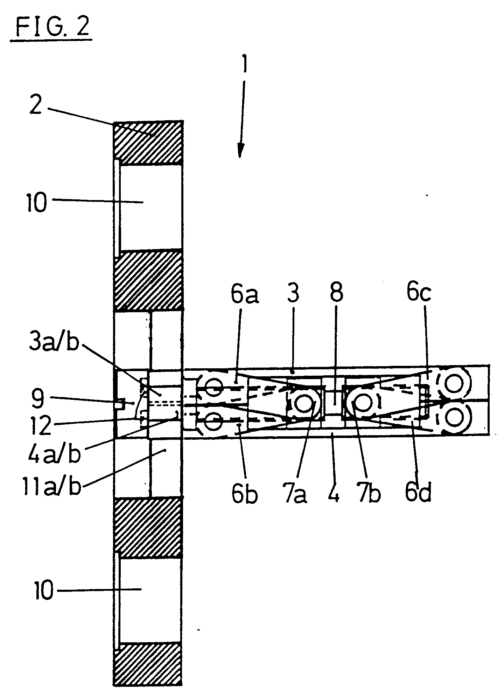

In **Fig. 2** sind dieselben Bezugszeichen wie in **Fig. 1** verwendet. Darin ist die Positionier- und Stützvorrichtung 1 in derselben Darstellung wie in **Fig. 1** dargestellt, wobei allerdings die Stützplatten 3 bzw. 4 auf den kleinsten Abstand zusammengefahren sind.

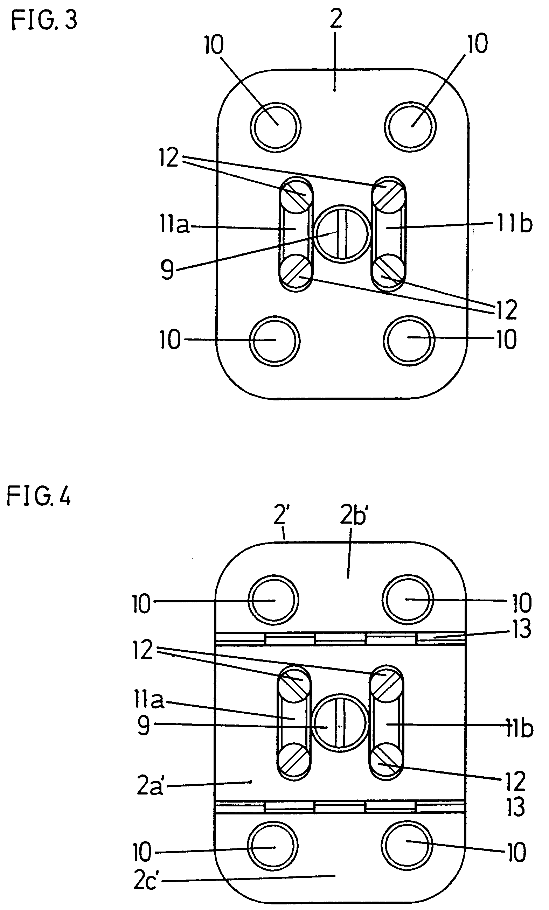

**Fig. 3** zeigt eine Ansicht der Trägerelement 2 von der Betätigungsseite aus. Es finden dieselben Bezugszeichen wie in den **Fig. 1** und **2** Verwendung.

Deutlich ist die Anordnung der Befestigungsbohrungen 10 sowie der Führungsschlitze 11a und 11b in der Trägerelement 2 erkannbar. In den Führungsschlitzen sind die Führungszapfen 3a und b und 4a und b geführt, die allerdings durch die Werkzeugansätze der eingeschraubten Fixierungsschrauben 12 verdeckt sind. Im Zentrum der Trägerelement 2 befindet sich der Werkzeugansatz 9 der in dieser Darstellung nicht sichtbaren Betätigungsschraube 8.

In **Fig. 4** ist eine Trägerelement 2 in derselben Ansicht wie die Trägerelement 2 in **Fig. 3** dargestellt. Es finden dieselben Bezugszeichen wie in **Fig. 3** Verwendung, soweit sie dieselben Bestandteile betreffen.

Die Trägerelement 2 besteht aus drei Teilstücken 2a', 2b' und 2c'. Die Teilstücke 2b' und 2c' sind über Scharniere 13 gelenkig mit dem Teilstück 2a' verbunden.

Bei der Implantation einer erfindungsgemäßen Positionier- und Stützvorrichtung zur Fusionierung von zwei Wirbelkörpern wird zunächst operativ ein ventraler Zugang zur Wirbelsäule geschaffen. Als nächstes wird die degenerierte Bandscheibe ausgeräumt. Hierbei wird zweckmäßigerweise - soweit möglich - die Wirbelsäule fixiert, um Schädigungen des Rückenmarks auszuschließen.

Die Stützplatten 3 und 4 der Positionier- und Stützvorrichtung werden mittels der Betätigungsschraube 8 vollständig zusammengefahren, wie in **Fig. 2** dargestellt. Nunmehr werden die zusammengefahrenen Stützplatten 3 und 4 von ventral zwischen die Stützkörper der benachbarten Wirbel eingesetzt, zwischen denen zuvor die Bandscheibe entfernt worden ist.

Das Trägerelement 2 wird ventral an die benachbarten Wirbelkörper angelegt. Anschließend wird ein passendes Werkzeug auf den Werkzeugansatz 9 der Betätigungsschraube 8 angesetzt und die Betätigungsschraube 8 nach rechts, d.h. im Uhrzeigersinn, betätigt. Dabei bewegen sich die Muttern 7a und b auseinander, wodurch über die Kniehebel 6a, b, c und d die Stützplatten 3 und 4 auseinandergespreizt werden.

Die Betätigungsschraube 8 wird in der beschriebenen Weise soweit verdreht, bis die Stützplatten 3 und 4 mit dem erforderlichen Anpreßdruck gegen die benachbarten Wirbelkörper gepreßt werden. Diese Anpassung ist sehr feinfühlig möglich, da das Scherenhubgestänge 5 gegenüber der Betätigung der Betätigungsschraube 8 hoch untersetzt ist. Dadurch läßt sich außerdem mit nur geringem Krafteinsatz des operierenden Chirurgen eine ausreichende Andruckkraft auf die Stützplatten 3 und 4 ausüben. In der gespreizten Stellung werden die Fixierungsschrauben 12 fest in die Führungszapfen 3a und b sowie 4a und b eingeschraubt. Dadurch wird deren Position in den Führungsschlitzen 11a und b fixiert. Somit wird auch bei starken Belastungen, beispielsweise beim Laufen und Springen auftretende Schlag- und Rüttelbewegungen, eine Lösung der Betätigungsschraube 8 unterbunden. Die Stützplatten 3 und 4 werden somit auch langfristig nicht zusammengedrückt.

Nach Anpassung der Stützplatten 3 und 4 wird die Trägerelement 2 durch die Befestigungsbohrungen 10 mittels Knochenschrauben mit den Wirbelkörpern verschraubt. Damit ist der operative Eingriff der Wirbelfusionierung abgeschlossen.

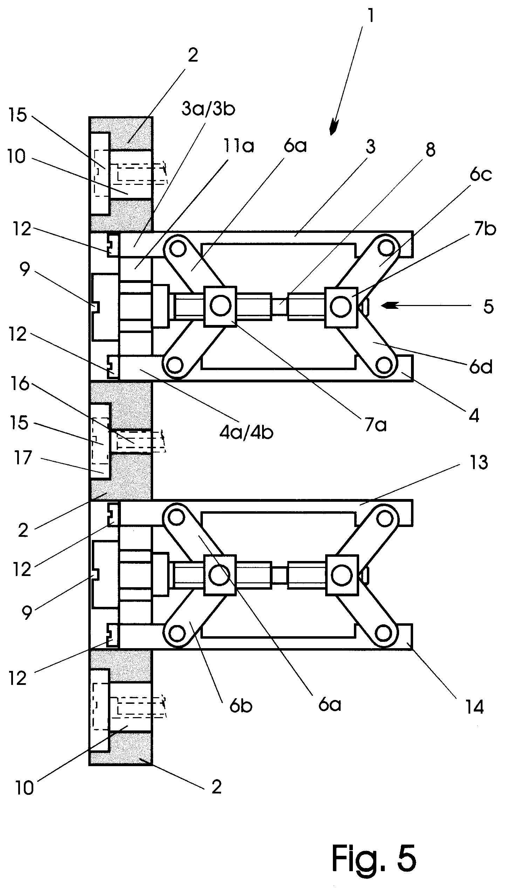

Die in **Fig. 5** dargestellte erfindungsgemäße Positionier- und Stützvorrichtung weist ein Trägerelement 2 und zwei Stützplattenpaare auf, deren Stützplatten 3; 4 und 13; 14 mit Abstand in etwa parallel zueinander angeordnet sind und von dem einstückig ausgebildeten Trägerelement 2 in der dargestellten Ausführungsform etwa rechtwinklig abstehen. Es werden dieselben Bezugszeichen wie in **Fig. 1** und **2** verwendet.

Die Stützplatten 3; 4 und 13; 14 sind jeweils, wie bereits zu **Fig. 1** beschrieben, mit dem Scherenhubgestänge 5 miteinander verbunden und an dem Tägerelement 2 angeordnet.

An den dem Trägerelement 2 zugewandten Seiten sind die Stützplatten 3; 4; 13; 14 jeweils mit zwei Führungszapfen 3a und b bzw. 4a und b versehen. Diese werden wie in **Fig. 2** dargestellt, längsverschiebbar in Führungsschlitzen 11a und b geführt, welche senkrecht verlaufend in das Trägerelement 2 eingebracht sind.

Das Trägerelement 2 ist an seinen Längsseiten mit langlochförmig ausgebildeten Befestigungsbohrungen 10 versehen und weist zwischen den Stützplattenpaaren eine zentral angeordnete Durchgangsbohrung 16 auf. Die Befestigungsbohrungen 10 und die Durchgangsbohrung 16 sind mit Aussparungen 17 zur Aufnahme der Schraubenköpfe der Befestigungsschrauben 15 versehen, wodurch ein bündiger Abschluß mit der Oberfläche des Trägerelementes 2 gegeben ist.

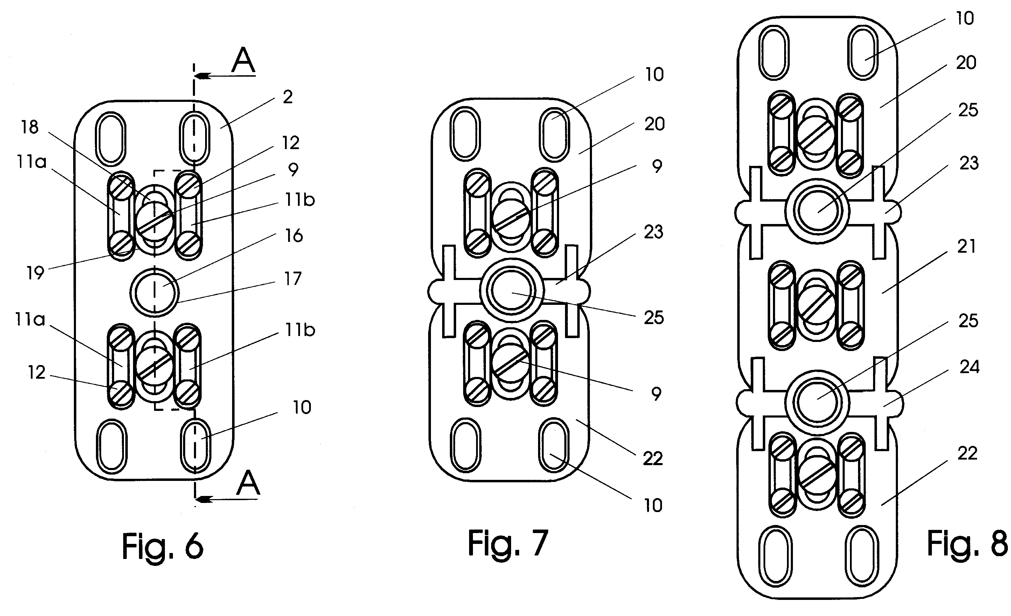

Bei der in **Fig. 7** dargestellten Ausführungsform der aus mehreren Stützplattenpaaren bestehenden Positionier- und Stützvorrichtung wird das Trägerelement 2, an das die Stützplatten 3; 4 und 13; 14, wie in **Fig. 5** beschrieben, verstell- und einstellbar angelenkt sind, durch die Trägerelementteile 20 und 21 gebildet. Beide Trägerelementteile 20; 21 sind durch ein Scharnier 23 gelenkig miteinander verbunden, wobei das Scharnier 23 in einem Abschnitt zwischen den aus den Stützplatten 3; 4 und 13; 14 bestehenden Stützplattenpaaren angeordnet ist. Es ist eindeutig zu erkennen, daß das Stützplattenpaar mit den Stützplatten 3 und 4 an dem Trägerelementteil 20 und das Stützplattenpaar mit den Stützplatten 13 und 14 am Trägerelementteil 21 angebracht ist.

Die Trägerelementteile 20; 21 sind entsprechend der Ausführung der Positionier- und Stützvorrichtung nach **Fig. 1, 2, 5** und **6** ausgebildet und verfügen über zwei vertikal angeordnete, parallel zueinander verlaufende Führungsschlitze 11a; 11b und eine mit einer Ausnehmung 19 versehene Nut 18, durch die die Stellschraube 8 zur Einstellung der Stützplatten 3; 4 bzw. 13; 14 hindurchgeführt ist.

Das Scharnier 23 und die Verbindung zwischen den Trägerelementteilen 20; 21 und dem Scharnier 23 sind so ausgebildet, daß die Positionier- und Stützvorrichtung auf der gegenüber den Wirbelkörpern abgewandten Seite eine einheitliche, ebene und bündige Oberfläche aufweist.

Endseitig sind beide Trägerelementteile 20; 21 mit nutförmigen Befestigungsbohrungen 10 versehen, die zusammen mit der zentralen Durchgangsbohrung 25, die im Scharnier 23 angeordnet ist, zur Fixierung und Befestigung der Positionier- und Stützvorrichtung an den Wirbelkörpern dienen.

In **Fig. 8** ist ein Beispiel für eine Positionier- und Stützvorrichtung aufgezeigt, die aus drei durch Scharniere 23; 24 gelenkig miteinander verbundenen Teilelementen zusammengesetzt ist. Die aus den Trägerelementteilen 20; 21; 22 mit jeweils einem Stützplattenpaar bestehenden Teilelemente sind entsprechend den **Fig. 1, 2, 3, 5, 6** und **7** ausgebildet.

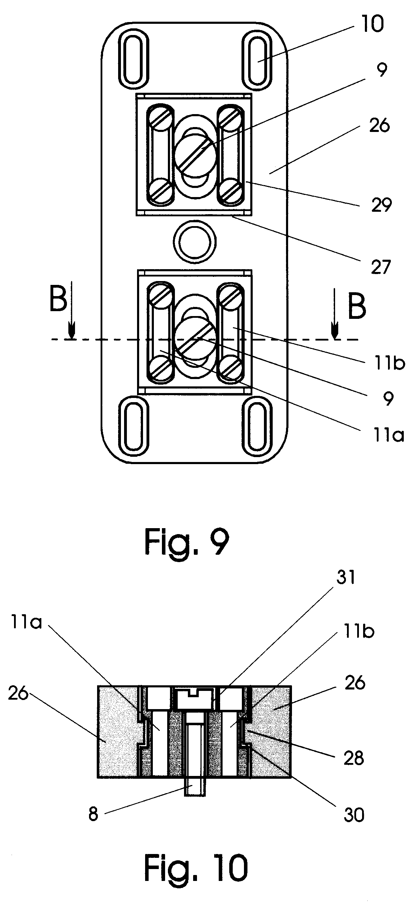

Eine weitere mögliche Ausführungsform der mehrere Stützplattenpaare aufweisenden Positionier- und Stützvorrichtung ist in **Fig. 9** und **10** schematisch dargestellt. Die Positionier- und Stützvorrichtung besitzt wiederum ein einstückig ausgebildetes Trägerelement 26 entsprechend den Ausführungen zu **Fig. 1** und **5** und ist in bereits beschriebener Art und Weise mit an seinen Enden angeordneten Befestigungsbohrungen 10 und einer zentralen Durchgangsbohrung 31 versehen.

Entsprechend den Abständen benachbarter Wirbelkörper sind in dem Trägerelement 26 Ausschnitte 27 eingebracht und in den vertikal verlaufenden Seiten dieser Ausschnitte 27 Führungsschienen 28 ausgeformt.

Die Schienen 28 dienen zur vertikal einstellbaren Aufnahme und Führung von Positionier- und Stützvorrichtungen, die jeweils über ein Stützplattenpaar mit den Stützplatten 3; 4 und einem Einstellmechanismus 6a - 6b; 7a; 7b; 8 und 9 verfügen und entsprechend **Fig. 1, 2, 5** und **6** ausgebildet sind.

In den vertikalen Stirnseiten des Trägerelementes 29 sind Führungsnuten 30 eingebracht, die in Zusammenwirken mit den Schienen 28 im Trägerelement 26 zur Aufnahme und Positionierung des mit Stützplattenpaaren ausgestatteten Trägerelementes 29 dienen und gleichzeitig eine zusätzliche Verstellung und Einstellung der Positionier- und Stützvorrichtung ermöglichen. Die Fixierung und endgültige Festlegung der Vorrichtung an der Wirbelsäule respektive an den Wirbelkörpern erfolgt in der bereits dargelegten Art und Weise.

Von diesem Ausführungsbeispiel ausgehend ist auch eine solche Lösung für eine Positionier- und Stützvorrichtung zur Fusionierung von 3 Wirbelkörpern praktikabel, die entsprechend **Fig. 3** ausgebildet ist, das Trägerelement jedoch keine Befestigungsbohrungen aufweist und dementsprechend kleiner ausgeführt ist. Zu ihrer Lagefixierung wird eine mit endseitigen und erforderlichenfalls zentralen Befestigungsbohrungen ausgestattete Sicherungsplatte auf das Trägerelement aufgesetzt und mit den Wirbelkörpern verschraubt. Die Sicherungsplatte ist an der den Trägerelementen der Positionier- und Stützvorrichtung zugewandten Seite mit einer Aussparung versehen, wodurch eine flächige Anlage der Sicherungsplatte an die betreffenden mit der Sicherungsplatte zu verschraubenden Wirbelkörper gewährleistet ist.

Bei der Implantation einer erfindungsgemäßen sogenannten "zweistöckigen" Positionier- und Stützvorrichtung wird operativ zunächst wie bei der Fusionierung von zwei Wirbelkörpern vorgegangen.

Die Stützplattenpaare mit den Stützplatten 3; 4 und 13; 14 werden in vollständig zusammengefahrener Form von ventral in die ausgeräumten Zwischenwirbelräume eingesetzt und das Trägerelement 2 sowie die Trägerelementteile 20 und 21 ventral an die benachbarten drei Wirbelkörper angelegt. Danach werden das Trägerelement 2 und die Trägerelementteile 20 und 21 durch die zentrale Befestigungsbohrung 16 bzw. 25 mittels einer Schraube 15 am mittleren Wirbelkörper grob fixiert.

Das Trägerelement 2 bzw. die Trägerelementteile 20; 21 der Positionier- und Stützvorrichtungen nach **Fig. 6** oder **7** sind mit dem Einsetzen der Stützplattenpaare bereits ventral an die benachbarten Wirbelkörper angelegt. Das Spreizen, Positionieren und Fixieren der Stützplattenpaare erfolgt ebenso wie beim Fusionieren von zwei Wirbelkörpern.

Nach der Anpassung der Stützplatten 3; 4 und 13; 14 werden das Trägerelement 2 bzw. die Trägerelementteile 20; 21 durch die Befestigungsbohrungen 10 mittels Schrauben 15 mit den Wirbelkörpern verschraubt und die Schraube 15 in den zentralen Befestigungsbohrungen 16; 25 endgültig fixiert. Damit ist der operative Eingriff der Fusionierung von drei Wirbelkörpern abgeschlossen.

### Bezugszeichen

- 2/2': Trägerelement
- 2a'/b'/c': Teilstücke
- 3: Stützplatte
- 3a/b: Führungszapfen
- 4: Stützplatte
- 4a/b: Führungszapfen
- 5: Scherenhubgestänge
- 6a/b: Kniehebel
- 6c/d: Kniehebel
- 7a/b: Muttern
- 8: Betätigungsschraube
- 9: Werkzeugansatz
- 10: Befestigungsbohrungen
- 11a/b: Führungsschlitze
- 12: Fixierungsschrauben
- 13/14: Stützplatte
- 15: Schraube
- 16: Durchgangsbohrung
- 17: Aussparung
- 18: Nut
- 19: Ausnehmung
- 20/21/22: Trägerelementteile
- 23: Scharnier
- 24: gelenkige Verbindung
- 25: Durchgangsbohrung
- 26: Trägerelement
- 27: Ausschnitte
- 28: Führungsschiene
- 29: Trägerelement
- 30: Führungsnut
- 31: Durchgangsbohrung

## Patentansprüche

1. Positionier- und Stützvorrichtung für die Wirbelsäule, mit einem im wesentlichen plattenförmigen Trägerelement, welches ventral an aufeinanderfolgenden Wirbeln festlegbar ist, und mit Abstand zueinander angeordneten Stützplatten, die an der gegen die Wirbelsäule gerichteten Seite des Trägerelementes angeordnet sind und deren Abstand voneinander einstellbar ist, **dadurch gekennzeichnet**, daß zwischen Stützplatten (3, 4, 13, 14) ein Scherenhubgestänge (5) mit einer Betätigungsschraube (8) zur Spreizung der Stützplatten (3, 4, 13, 14) angeordnet ist, wobei die Betätigungsschraube (8) in dem Trägerelement (2) drehbar gelagert ist und an der den Stützplatten (3,4,13,14) gegenüberliegenden Seite des Trägerelementes (2) einen Werkzeugansatz (9) aufweist.

2. Positionier- und Stützvorrichtung nach Anspruch 1, **dadurch gekennzeichnet**, daß an einem Trägerelement (2) mindestens zwei Stützplattenpaare aus jeweils zwei mit Abstand zueinander angeordneten Stützplatten (3, 4; 13, 14) angeordnet sind, wobei der Abstand der Stützplattenpaare unabhängig vom Abstand der Stützplatten (3, 4, 13, 14) einstellbar ist.

3. Positionier- und Stützvorrichtung nach Anspruch 1 oder 2, **dadurch gekennzeichnet**, daß das Trägerelement (2) aus gelenkig miteinander verbundenen Trägerelementteilen (20, 21, 22) und das Trägerelement (2') aus miteinander gelenkig verbundenen Teilstücken (2a', 2b', 2c') gebildet wird, wobei die gelenkige Verbindung (23, 24) jeweils im Bereich zwischen den Stützplattenpaaren an den Trägerelementteilen (20, 21, 22) angeordnet ist und vorzugsweise durch ein Scharnier gebildet wird, das eine zentrale Durchgangsbohrung (25) aufweist.

4. Positionier- und Stützvorrichtung nach einem der Ansprüche 1 bis 3, **dadurch gekennzeichnet**, daß die Stützplatten (3, 4, 13, 14) in gespreizter Stellung fixierbar und/oder an dem Trägerelement (2) und den Trägerelementteilen (20, 21, 22) geführt sind und gegebenenfalls gegeneinander und/oder gegenüber dem Trägerelement (2) und den Trägerelementteilen (20, 21, 22) kippbar sind.

5. Positionier- und Stützvorrichtung nach einem der Ansprüche 1 bis 4, **dadurch gekennzeichnet**, daß das Trägerelement (2, 2') und die Trägerelementteile (20, 21, 22) Führungsschlitze (11a, 11b) aufweisen, in denen an den Stützplatten anbringbare Zapfen (3a, 3b, 4a, 4b) geführt sind.

6. Positionier- und Stützvorrichtung nach einem der Ansprüche 1 bis 4, **dadurch gekennzeichnet**, daß das Trägerelement (2) und die Trägerelementteile (20, 21, 22) an den Wirbelkörpern zwischen den an ihnen angeordneten Stützplattenpaaren fixierbar sind.

7. Positionier- und Stützvorrichtung nach Anspruch 6, **dadurch gekennzeichnet**, daß das Trägerelement (2) und die Trägerelementteile (20, 21, 22) eine zentral zwischen Stützplattenpaaren angeordnete Befestigungsbohrung (16) aufweisen.

8. Positionier- und Stützvorrichtung nach Anspruch 6 oder 7, **dadurch gekennzeichnet**, daß das Trägerelement (2) und die Trägerelementteile (20, 22) oben und unten langlochartig ausgebildete Befestigungsbohrungen (10) zur Fixierung an den Wirbelkörpern aufweisen.

9. Positionier- und Stützvorrichtung nach Anspruch 1 oder 2, **dadurch gekennzeichnet**, daß das Trägerelement (26) zwei voneinander beabstandete Ausschnitte (27) aufweist, in deren vertikal verlaufenden Seiten eine Führungsschiene (28) ausgeformt ist, die in eine Führungsnut (30) eingreift, welche in den Seiten eines Trägerelementes (29) einer Stützvorrichtung vorgesehen ist.

10. Positionier- und Stützvorrichtung nach Anspruch 1 oder 2, **dadurch gekennzeichnet**, daß sie aus mehreren in Wirbelkörperzwischenräumen positionierbaren Stützvorrichtungen, bestehend aus einem Trägerelement (29), einem Stützplattenpaar und einem Scherenhubmechanismus, und einer aufschraubbaren Sicherungsplatte zur Lagesicherung gebildet ist.

11. Positionier- und Stützvorrichtung nach Anspruch 10, **dadurch gekennzeichnet**, daß die Sicherungsplatte auf der den Stützvorrichtungen zugewandten Seite Aussparungen zur bündigen Aufnahme der Trägerelemente aufweist.

12. Positionier- und Stützvorrichtung nach Anspruch 1, **dadurch gekennzeichnet**, daß die Stützplatten (3, 4, 13, 14) mit Durchbrüchen versehen sind und/oder eine aufgerauhte Oberfläche aufweisen.

## Claims

1. Positioning and support device for the spinal column, with a plate serving as the basic fixation element, which is attached ventral to the discs, and with distractible supportive plates that are located some distance from one another on the side of the fixation element against the spinal column; the distance can be adjusted, characterized in that, between the supportive plates (3, 4, 13, 14) there is a scissors-type jack (5) with a set screw (8) to spread the supportive plates (3, 4, 13, 14), whereby the set screw (8) is located in the fixation plate (2), is rotatable and has its head on the side of the fixation plate (2) opposite the supportive plates (3, 4, 13, 14).

2. Positioning and support device as claimed in Claim 1, characterized by a fixation plate (2) at least two pairs of support plates of each two support plates separated from one another (3, 4, 13, 14), whereby the distance of the supporting plates pairs can be adjusted independently of distance of the supportive plates.

3. Positioning and support device as claimed in Claims 1 or 2, characterized by the fixation plate (2) consisting of articulated parts that are connected with one another (20, 21, 22) and the fixation plate (2') is formed of articulated, connected sections (2a', 2b', 2c') whereby the hinged connection (23, 24) is attached to the fixation plates (20, 21, 22) in the area between the support plate pairs and is preferably made of a hinge with a central throughhole (25).

4. Positioning and support device as claimed in Claims 1 to 3, characterized by the supportive plates (3, 4, 13, 14) being fixed in spread position and/or attached to the fixation plate or sections of the fixation plate (20, 21, 22) and are inclinable with relation to one another and/or the fixation plate (2) and the fixation sections (20, 21, 22).

5. Positioning and support device as claimed in Claims 1 to 4, characterized by the fixation plate (2, 2') and the fixation sections (20, 21, 22) have guide slots(11a, 11b) from which pins (3a, 3b, 4a, 4b) lead to the supportive plates.

6. Positioning and support device as claimed in Claims 1 to 4, characterized by the fixation plate (2, 2') and the fixation sections (20, 21, 22) are attachable to the discs between the supportive plate pairs attached to them.

7. Positioning and support device as claimed in Claim 6, characterized by the fixation plate (2) and the fixation sections (20, 21, 22) having a central hole (16) for attachment in the middle between the support plate pairs.

8. Positioning and support device as claimed in Claims 6 or 7, characterized by the fixation plate (2) and the fixation sections (20, 22) having oblong holes (10) at the top and bottom for attachment to the discs.

9. Positioning and support device as claimed in Claims 1 or 2, characterized by the fixation plate (2) having two separate sections (27), the vertical sides of which have tracks (28), with a guide groove (30) that is in the side of the fixation plate (29) of a support device.

10. Positioning and support device as claimed in Claims 1 or 2, characterized by several supportive devices locatable in the spaces between discs, consisting of a fixation plate (29), a pair of supporting plates and a scissors-type jack mechanism and safety plate which can be attached for securing the position.

11. Positioning and support device as claimed in Claim 10, characterized by sides of the safety plate facing the support plates having grooves for the attachment of the fixation plates.

12. Positioning and support device as claimed in Claim 1, characterized by the supporting plates (3, 4, 13, 14) having breaks and/or a rough surface.

## Revendications

1. Dispositif de soutien et de positionnement pour la colonne vertébrale muni d'un élément de support essentiellement en forme de plate-forme susceptible d'être fixé ventralement à des vertèbres successives, et muni de plaques de soutien disposées avec un écart les unes par rapport aux autres et disposées à la face de l'élément de support orientée vers la colonne vertébrale et dont l'écart les unes par rapport aux autres est susceptible d'être ajusté, **caractérisé par le fait qu'**un système de tiges de levage en ciseau (5) muni d'une vis d'actionnement (8) destinée à écarter les plaques de soutien (3, 4, 13, 14) est disposé entre des plaques de soutien (3, 4, 13, 14), la vis d'actionnement (8) étant logée de façon susceptible de tourner dans l'élémént de support (2) et présentant à la face de l'élément de support (2) opposée aux plaques de soutien (3, 4, 13, 14) un embout pour un instrument (9).

2. Dispositif de soutien et de positionnement selon la revendication 1, **caractérisé par le fait qu'**au moins deux paires de plaques de soutien se composant chaque fois de deux plaques de soutien (3, 4 ; 13, 14), disposées avec un écart l'une par rapport à l'autre, sont disposées à un élément de support (2), l'écart des paires de plaques de soutien étant susceptible d'être ajusté indépendamment de l'écart des plaques de soutien (3, 4 ; 13, 14).

3. Dispositif de soutien et de positionnement selon la revendication 1 ou 2, **caractérisé par le fait que** l'élément de soutien (2) est formé de parties d'élément de support (20, 21, 22) raccordées les unes aux autres de façon susceptible de s' articuler et que l'élément de support (2') est formé de tronçons (2a', 2b', 2c') raccordés les uns aux autres de façon susceptible de s'articuler, le raccord articulé (23, 24) étant disposé aux parties d'élément de support (20, 21, 22) chaque fois dans le domaine situé entre les paires de plaques de soutien et étant formé de préférence par une charnière qui présente un perçage central de passage (25).

4. Dispositif de soutien et de positionnement selon l'une des revendications 1 à 3, **caractérisé par le fait que** les plaques de soutien (3, 4, 13, 14) sont susceptibles d'être fixées en position écartée et/ou sont guidées à l'élément de support (2) et aux parties d'élément de support (20, 21, 22) et sont, le cas échéant, susceptibles de basculer l'une vers l'autre et/ou par rapport à l'élément de support (2) et aux parties d'élément de support (20, 21, 22).

5. Dispositif de soutien et de positionnement selon l'une des revendications 1 à 4, **caractérisé par le fait que** l'élément de support (2, 2') et les parties d'élément de support (20, 21, 22) présentent des fentes de guidage (11a, 11b) dans lesquelles des tourillons (3a, 3b, 4a, 4b) susceptibles d'être installés sont guidés aux plaques de soutien.

6. Dispositif de soutien et de positionnement selon l'une des revendications 1 à 4, **caractérisé par le fait que** l'élément de support (2) et les parties d'élément de support (20, 21, 22) sont susceptibles d'être fixés aux vertèbres entre les paires de plaques de soutien disposées à celles-ci.

7. Dispositif de soutien et de positionnement selon la revendication 6, **caractérisé par le fait que** l'élément de support (2) et les parties d'élément de support (20, 21, 22) présentent un perçage de fixation (16) disposé centralement entre des paires de plaques de soutien.

8. Dispositif de soutien et de positionnement selon la revendication 6 ou 7, **caractérisé par le fait que** l'élément de support (2) et les parties d'éléments de support (20, 22) présentent des perçages de fixation (10) développés en haut et en bas à la façon d'un trou allongé et destinés à fixer aux vertèbres.

9. Dispositif de soutien et de positionnement selon la revendication 1 ou 2, **caractérisé par le fait que** l'élément de support (26) présente deux découpes écartées l'une de l'autre dans les faces desquelles s'étendant verticalement il est formé un rail de guidage (28) mordant dans une rainure de guidage (30), rainure de guidage étant prévue dans les faces d'un élément de support (29) d'un dispositif de soutien.

10. Dispositif de soutien et de positionnement selon la revendication 1 ou 2, **caractérisé par le fait qu'**il est formé de plusieurs dispositifs de soutien susceptibles d'être positionnés dans des espaces intervertébrals se composant d'un élément de support (29), d'une paire de plaques de soutien et d'un mécanisme de levage en ciseau et d'une plaque de sûreté susceptible d'être vissée dessus et destinée à fixer.

11. Dispositif de soutien et de positionnement selon la revendication 10, **caractérisé par le fait que** la plaque de sûreté présente sur la face tournée vers les dispositifs de soutien des creux destinés à réceptionner à même hauteur les éléments de support.

12. Dispositif de soutien et de positionnement selon la revendication 1, **caractérisé par le fait que** les plaques de soutien (3, 4, 13, 14) sont pourvues de percements et/ou présentent une surface rendue rugueuse.
